# EUROPÄISCHE PATENTSCHRIFT

(11) **EP 1 368 016 B1**
(45) Veröffentlichungstag und Bekanntmachung des Hinweises auf die Patenterteilung: **19.04.2006**
(21) Anmeldenummer: 01994735.7
(22) Anmeldetag: 27.11.2001
(51) Int. Cl.: A61K 31/135, A61K 31/195, A61K 9/10, A61K 47/44

(54) **PARENTAL APPLIZIERBARE DARREICHUNGSFORMEN ENTHALTEND EINE SUSPENSION DES SALZES AUS TRAMADOL UND DICLOFENAC**
PARENTERAL DOSAGE FORMS COMPRISING A SUSPENSION OF TRAMADOL SALT AND DICLOFENAC SALT
FORMES D'ADMINISTRATION PARENTERALE DE SUBSTANCES CONTENANT UNE SUSPENSION DU SEL DE TRAMADOL ET DU SEL DE DICLOFENAC

(30) Priorität: 28.11.2000 DE 10059020
(43) Veröffentlichungstag der Anmeldung: 10.12.2003
(73) Patentinhaber: Grünenthal GmbH, 52078 Aachen (DE)
(72) Erfinder: BARTHOLOMÄUS, Johannes, 52080 Aachen (DE); KUGELMANN, Heinrich, 52068 Aachen (DE)
(74) Vertreter: Kutzenberger, Helga
(86) Internationale Anmeldenummer: PCT/EP2001/013789
(87) Internationale Veröffentlichungsnummer: WO 2002/043723

(56) Entgegenhaltungen:
- WO-A-00/72827
- US-A- 4 016 273
- US-A- 5 283 067
- US-A- 5 336 691
- US-A- 5 958 379
- GRONING R: "Computer-controlled drug release from small-sized dosage forms" JOURNAL OF CONTROLLED RELEASE, ELSEVIER SCIENCE PUBLISHERS B.V. AMSTERDAM, NL, Bd. 48, Nr. 2-3, 13. Oktober 1997 (1997-10-13), Seiten 185-193, XP004125855 ISSN: 0168-3659

## Beschreibung

Die vorliegende Erfindung betrifft parenteral applizierbare Darreichungsformen enthaltend eine Suspension des Salzes aus den Wirkstoffen Tramadol und Diclofenac.

Der pharmazeutische Wirkstoff Tramadol wird häufig in Form seines Hydrochlorids - (1RS,2RS)-2-[(Dimethylamino)methyl]-1-(3-methoxyphenyl)-cyclohexanol-hydrochlorid - als Analgetikum zur Bekämpfung mittelstarker bis starker Schmerzen eingesetzt.

Bei manchen Patienten ist eine zufriedenstellende Schmerzbekämpfung nur über eine parenterale Verabreichung der Schmerzmittel zu erreichen, beispielsweise wenn der Patient aufgrund seiner körperlichen Beeinträchtigung nicht oder nur schlecht in der Lage ist, ein Schmerzmittel oral einzunehmen.

Ein Nachteil der parenteralen Applikation von Tramadolhydrochlorid ist die relativ schnelle Metabolisierung des Wirkstoffes aus den bisher bekannten Darreichungsformen, so daß nach seiner Verabreichung eine länger anhaltende analgetische Wirkung ohne Erhöhung der Dosierung nicht zu erzielen ist. Eine solche Erhöhung ist jedoch nicht wünschenswert, da damit auch das Risiko unerwünschter Begleiterscheinungen zunimmt.

Die der vorliegenden Erfindung zugrundeliegende Aufgabe bestand daher darin, eine Darreichungsform des Wirkstoffes Tramadol zur parenteralen Verabreichung zur Verfügung zu stellen, aus der dieser Wirkstoff verzögert metabolisiert wird.

Erfindungsgemäß wird diese Aufgabe durch die Bereitstellung parenteral applizierbarer Darreichungsformen gelöst, die eine Suspension des Wirkstoffsatzes aus Tramadol und Diclofenac enthalten.

US 5,336,691 offenbart Zubereitungen enthaltend Tramadol und Acetominophen, die u.a. für die parenterale Verabreichung vorgesehen sind. Die parenterale Darreichungsformen enthalten den Wirkstoff Tramadol in gelöster Form.

US 5,283,067 beschreibt parenteral applizierbare Darreichungsformen enthaltend Suspensionen des Natriumsalzes von Diclofenac.

Zur Herstellung des Wirkstoffsalzes aus Tramadol und Diclofenac wird bevorzugt ein sehr gut wasserlösliches Salz des Tramadols mit einem sehr gut wasserlöslichen Salz des Diclofenacs umgesetzt. Als Salz des Tramadols wird bevorzugt Tramadolhydrochlorid, als Salz des Diclofenacs bevorzugt dessen Natriumsalz eingesetzt. Das so erhaltene Wirkstoffsalz aus Tramadol und Diclofenac kann nach verschiedenen, dem Fachmann bekannten Methoden isoliert und gegebenenfalls gereinigt werden.

In einer bevorzugten Ausführungsform der vorliegenden Erfindung weisen mindestens 95 % der suspendierten Salzpartikel der erfindungsgemäßen, parenteral applizierbaren Darreichungsformen einen Partikeldurchmesser im Bereich von ≤ 50 µm, vorzugsweise ≤ 30 µm, besonders bevorzugt ≤ 5 µm auf.

Die Partikeldurchmesser der suspendierten Salzpartikel werden über eine Streulichtmessung in einem Coulter® LS 230 Laserpartikelanalysator mit HFM- und MVM-Modul (Beckman-Coulter Electronics GmbH, Krefeld, Germany) bestimmt.

Das Suspensionsmedium der erfindungsgemäßen, parenteral applizierbaren Darreichungsformen kann hydrophob oder hydrophil sein.

Eine Ausführungsform der vorliegenden Erfindung besteht daher darin, daß das Wirkstoffsalz aus Tramadol und Diclofenac in einem hydrophoben pharmazeutisch verträglichen Suspensionsmedium suspendiert ist. Vorzugsweise kann dieses hydrophobe Suspensionsmedium auf pharmazeutisch verträglichen synthetischen, halbsynthetischen oder natürlichen Ölen oder Mischungen aus wenigstens zwei dieser Öle basieren.

Als synthetische, halbsynthetische oder natürlichen Öle können vorzugsweise mittelkettige Triglyceride mit einer Kettenlänge von C₈ bis C₁₀ im Carbonsäure-Teil, Sojaöl, Sesamöl, Erdnußöl, Olivenöl, Kokosöl, Rizinusöl, Sonnenblumenöl, Distelöl oder die entsprechenden, hydrierten Öle oder Mischungen aus wenigstens zwei der vorstehend genannten Öle eingesetzt werden. Besonders bevorzugt wird Rizinusöl eingesetzt. Gegebenenfalls können diese Öle auch mit physiologisch verträglichen Antioxidantien, vorzugsweise Tocopherolen und/oder deren Ester, butyliertem Hydroxyanisol oder butyliertem Hydroxytoluol ausgerüstet sein, vorzugsweise in Mengen von 0,001 bis 0,1 Gew.-%, bezogen auf das Suspensionsmedium.

Für die erfindungsgemäßen, parenteral applizierbaren Darreichungsformen des Wirkstoffsalzes kann auch ein physiologisch verträgliches hydrophiles Suspensionsmedium eingesetzt werden. Vorzugsweise basiert das hydrophile Suspensionsmedium auf Wasser.

Das physiologisch verträgliche hydrophile Suspensionsmedium kann neben Wasser weitere physiologisch verträgliche Hilfsstoffe enthalten. Vorzugsweise sind diese Hilfsstoffe pH-Regulatoren, Regulatoren zur Einstellung der Osmolalität, grenzflächenaktive Verbindungen, Viskositätsregulatoren, Peptisatoren, Puffer oder Konservierungsmittel.

Neben einem oder mehreren Regulatoren zur Einstellung der Osmolalität können die erfindungsgemäßen Darreichungsformen weiterhin einen oder mehrere Vertreter einer bis aller übrigen genannten Hilfsstoffklassen enthalten.

Sofern die erfindungsgemäßen parenteral applizierbaren Darreichungsformen physiologisch verträgliche grenzflächenaktive Verbindungen enthalten, werden bevorzugt Polyalkylenglykole, wie Polyethylenglykole, Polypropylenglykole oder Ethylenoxid-, Propylenoxid-Blockcopolymere, Phospholipide, Ether oder Ester von gesättigten oder ungesättigten Fettalkoholen oder Fettsäuren mit Polyalkylenglykolen, wie Polyethylenglykolen oder Polypropylenglykolen, Polysorbate, wie Mono-, Di-, oder Triester von gesättigten oder ungesättigten Fettsäuren, besonders bevorzugt Ölsäure, Laurinsäure, Palmitinsäure oder Stearinsäure, und Sorbitol und/oder seinem Anhydrid, die bis zu 20 Mol Ethylenoxid-Einheiten pro Mol Sorbitol bzw. Anhydrid aufweisen können, vorzugsweise Polyethoxysorbitanmonolaurat mit 20 Ethylenoxid-Einheiten, Polyethoxysorbitanmonolaurat mit 4 Ethylenoxid-Einheiten, Polyethoxysorbitanmonopalmitat mit 20 Ethylenoxid-Einheiten, Polyethoxysorbitanmonostearat mit 20 Ethylenoxid-Einheiten, Polyethoxysorbitanmonostearat mit 4 Ethylenoxid-Einheiten, Polyethoxysorbitantristearat mit 20 Ethylenoxid-Einheiten, Polyethoxysorbitanmonooleat mit 20 Ethylenoxid-Einheiten, Polyethoxysorbitanmonooleat mit 5 Ethylenoxid-Einheiten oder Polyethoxysorbitantrioleat mit 20 Ethylenoxideinheiten, oder ein Gemisch aus wenigstens zwei der vorstehend genannten grenzflächenaktiven Verbindungen eingesetzt.

Eine Vielzahl der entsprechenden Polysorbate wird unter dem Handelsnamen Tween® von der Firma UNIQEMA/ICI group of companies am Markt geführt.

Um das Risiko von Zell- und Gewebeschädigungen zu minimieren bzw. völlig auszuschließen, wird die Osmolalität, d.h. die Tonizität der parenteral zu applizierenden, erfindungsgemäßen Darreichungsformen bevorzugt so eingestellt, daß sie isotonisch oder zumindest annähernd isotonisch zu der physiologischen Osmolalität sind.
Bevorzugt wird daher die Osmolalität der erfindungsgemäßen, parenteral applizierbaren Darreichungsformen so eingestellt, daß sie im Bereich von 250 bis 400 mOsm/kg, besonders bevorzugt im Bereich von 260 bis 320 mOsm/kg und ganz besonders bevorzugt im Bereich von 280 bis 300 mOsm/kg liegt.

Bevorzugte Regulatoren zur Einstellung der Osmolalität sind wasserlösliche, physiologisch verträgliche Verbindungen wie anorganische Salze, z.B. Alkalisalze, bevorzugt Natriumchlorid, Zucker, z.B. Saccharose oder Dextrose, Zuckeralkohole, z.B. Mannitol, oder Polyalkylenglykole, z.B. Polyethylenglykole, vorzugsweise mit einem Molekulargewicht von 1000 bis 8000 g/mol. Es kann auch ein Gemisch aus wenigstens zwei Vertretern aus verschiedenen, vorstehend genannten Regulatorenklassen oder wenigstens zwei Vertretern aus einer Regulatorenklasse zur Einstellung der Osmolalität verwendet werden.

Gegebenenfalls kann ein Regulator auch zur Einstellung verschiedener Eigenschaften der erfindungsgemäßen Darreichungsformen eingesetzt werden. Beispielsweise kann eine grenzflächenaktive Verbindung auch zur Einstellung der Osmolalität verwendet werden.

Der pH-Wert der erfindungsgemäßen Darreichungsformen sollte vorzugsweise im Bereich von pH 5 bis pH 8 liegen, um weitere Risiken von Zell- und Gewebeschädigungen zu vermeiden.

Die erfindungsgemäßen, parenteral applizierbaren Darreichungsformen können auch physiologisch verträgliche Konservierungsmittel enthalten. Als solche eignen sich beispielsweise 1,1,1-Trichlor-2-methyl-2-propanol, Phenylethylalkohol, Sorbinsäure, Benzylalkohol, Alkylbenzyldimethylammoniumchlorid mit einer Kettenlänge von C₈ bis C₁₈ im Alkylteil, m-Kresol oder 4-Hydroxyalkylbenzoat, vorzugsweise 4-Hydroxymethylbenzoat oder 4-Hydroxypropylbenzoat. Es können auch Mischungen aus zwei oder mehreren der vorstehend genannten Konservierungsmittel zum Einsatz kommen.

In einer besonders bevorzugten Ausführungsform der vorliegenden Erfindung ist das Suspensionsmedium der erfindungsgemäßen Darreichungsformen Wasser, das als weitere Hilfsstoffe
a) 0,001 bis 1 Gew.-%, vorzugsweise 0,0015 bis 0,1 Gew.-%, besonders bevorzugt 0,005 bis 0,015 Gew.-% physiologisch verträgliche, grenzflächenaktive Verbindungen, vorzugsweise Polysorbate, besonders bevorzugt mit Ölsäure, Laurinsäure, Palmitinsäure oder Stearinsäure einfach verestertes und mit Polyethylenglykol, vorzugsweise mit Polyethylenglykol mit einem Molekulargewicht von 1000 bis 8000 g/mol, verethertes Sorbitol und/oder seine Anhydride
b1) 3 bis 10 Gew.-% wenigstens eines Osmolalitäts-Regulators ausgewählt aus der Gruppe Monosaccharide, Oligosaccharide und Zuckeralkohole, vorzugsweise Saccharose und/oder Mannitol, oder
b2) eine Mischung aus wenigstens einem der unter b1) genannten Osmolalitäts-Regulatoren in Mengen von 0,5 bis 5 Gew.-% und aus Polyethylenglykolen, vorzugsweise Polyethylenglykolen mit einem Molekulargewicht von 1000 bis 8000 g/mol, in Mengen von 10 bis 20 Gew.-%,
jeweils bezogen auf das gesamte Suspensionsmedium einschließlich der Komponenten a) und b1) oder a) und b2), enthält.

Das zu applizierende Volumen der erfindungsgemäßen parenteral applizierbaren Darreichungsformen beträgt vorzugsweise ≤ 5 ml, besonders bevorzugt ≤ 4 ml und ganz besonders bevorzugt ≤ 2 ml.

Vorzugsweise eignen sich die erfindungsgemäßen, parenteral applizierbaren Darreichungsformen zur intramuskulären oder subkutanen Applikation.

Die an den Patienten zu verabreichende Menge des Wirkstoffsalzes in den erfindungsgemäßen Darreichungsformen kann z.B. in Abhängigkeit vom Gewicht des Patienten, der Art oder der Stärke der Schmerzen variieren. Dem Fachmann ist aufgrund der Wirkung der Analgetika bekannt, in welchen Dosierungen das Wirkstoffsalz einzusetzen ist, damit die gewünschte Wirkung erzielt wird.

Die Herstellung der erfindungsgemäßen, parenteral applizierbaren Darreichungsformen kann nach üblichen, dem Fachmann bekannten Methoden erfolgen.

Vorzugsweise erfolgt die Herstellung der erfindungsgemäßen Darreichungsformen auf wäßriger Basis nach der nachstehend beschriebenen Methode.

Die zum Einsatz kommenden Hilfsstoffe werden bei Raumtemperatur oder gegebenenfalls unter Erwärmen in Wasser für Injektionszwecke gelöst. Sofern es es sich um flüssige Hilfsstoffe handelt, werden diese mit Wasser gemischt. Die so erhaltene Lösung bzw. Mischung wird anschließend unter Verwendung eines Filters, welcher Mikroorganismen zurückhält, steril filtriert. Üblicherweise beträgt die Porenweite dieses Filters 0,2 µm. Die Filtration kann gegebenenfalls auch vor der Zugabe der Hilfsstoffe erfolgen, jedoch sollte dann die weitere Herstellung der Darreichungsformen unter aseptischen Bedingungen erfolgen.

Anschließend wird das sterile Wirkstoffsalz aus Tramadol und Diclofenac unter aseptischen Bedingungen durch Rühren, vorzugsweise homogen in dem so erhaltenen hydrophilen Suspensionsmedium suspendiert und die so erhaltene Suspension anschließend in geeignete Behälter, vorzugsweise in Injektionsflaschen (vials) abgefüllt.

Die Herstellung der erfindungsgemäßen Darreichungsformen auf hydrophober Basis erfolgt vorzugsweise dadurch, daß zunächst das hydrophobe Suspensionsmedium durch Hitze sterilisiert wird und ggf. weitere Hilfsstoffe zugegeben werden.

Anschließend wird das sterile Wirkstoffsalz aus Tramadol und Diclofenac unter aseptischen Bedingungen durch Rühren, vorzugsweise homogen in dem so erhaltenen hydrophoben Suspensionsmedium suspendiert und die so erhaltene Suspension anschließend in geeignete Behälter, vorzugsweise in Injektionsflaschen (vials) abgefüllt.

Sofern die Herstellung der erfindungsgemäßen, parenteral applizierbaren Darreichungsformen nicht unter aseptischen Bedingungen erfolgt ist, kann ggf. eine Endsterilisation nach üblichen, dem Fachmann bekannten Methoden, beispielsweise durch Autoklavieren, vorgenommen werden. Vorzugsweise werden bereits die erfindungsgemäßen, parenteral applizierbaren Suspensionen unter aseptischen Bedingungen hergestellt.

Die erfindungsgemäßen, parenteral applizierbaren Darreichungsformen zeichnen sich unter anderem auch dadurch aus, daß sie über einen längeren Zeitraum lagerstabil sind, d.h. applizierbar bleiben. Bei den erfindungsgemäßen Darreichungsformen tritt auch bei monatelanger Lagerung keine irreversible Agglomeration auf.
Hierbei kann es vorteilhaft sein, kleine inerte Festkörper, wie z.B. Glaskugeln in das Suspensionsgefäß zu geben, um dadurch beim Schütteln der gelagerten Suspension eine homogene Resuspendierung schneller wieder zu erreichen.

Die erfindungsgemäßen parenteral applizierbaren Darreichungsformen haben ferner den Vorteil, daß der analgetische Wirkstoff Tramadol zur Schmerzbekämpfung länger zur Verfügung steht als bei einer parenteralen Arzneiform von Tramadolhydrochlorid. Dies ermöglicht auch bei Patienten, bei denen eine orale Therapie unter Verwendung dieser Wirkstoffe nicht in Frage kommt eine effektive Schmerzbekämpfung, ohne daß das Tramadol z.B. mehr als zweimal täglich an den Patienten verabreicht werden muß. Hierdurch wird für den Patienten eine effektive Schmerztherapie unter geringerer Belastung erreicht. Desweiteren ermöglicht die verzögerte Metabolisierung die Einstellung konstanter Serumspiegel des Wirkstoffes Tramadol im Blutserum. Hohe Wirkstoffkonzentrationen, wie sie bei herkömmlichen Darreichungsformen mit schneller Wirkstoffabgabe häufig auftreten sowie die damit gegebenenfalls verbundenen unerwünschten Begleiterscheinungen können so vollständig vermieden oder zumindest deutlich reduziert werden.

Die **Figur 1** zeigt die Konzentrationszeitverläufe des (+)-Tramadol-Enantiomeren für die erfindungsgemäßen Darreichungsformen sowie für eine entsprechende Tramadolhydrochlorid Darreichungsform im Blutserum von Hunden nach parenteraler Applikation.

Die **Figur 2** zeigt die Konzentrationszeitverläufe des (-)-Tramadol-Enantiomeren für die erfindungsgemäßen Darreichungsformen sowie für eine entsprechende Tramadolhydrochlorid Darreichungsform im Blutserum von Hunden nach parenteraler Applikation.

### Pharmakologische Untersuchungen:

5 Hunden (Rasse: Beagle, Züchter: Harlan-Winkelmann, Borchen, Deutschland) wurde jeweils eine erfindungsgemäße Darreichungsform enthaltend Tramadol-Diclofenac-Salz bzw. eine entsprechende Tramadolhydrochlorid-Darreichungsform parenteral verabreicht. Jeweils bei 0; 0,5; 1; 2; 4; 7, bzw. für die erfindungsgemäßen Darreichungsformen auch bei 24 Stunden nach der Applikation der jeweiligen Darreichungsform wurde jedem Tier eine Blutprobe entnommen.
Zwischen jeder Applikation der erfindungsgemäßen Darreichungsformen bzw. zwischen jeder Tramadolhydrochlorid-Darreichungsform wurde eine behandlungsfreie Zeit von wenigstens 14 Tagen eingehalten.

Die Blutproben wurden nach der Abnahme zentrifugiert, der feste Rückstand verworfen und das so erhaltene Blutserum bis zur Analyse bei einer Temperatur unterhalb von -20°C gelagert.

Jede Meßserie bestand aus jeweils acht Kalibrationsproben unterschiedlicher Konzentration, Qualitätskontrollproben mit jeweils zwei Proben in drei verschiedenen Konzentrationsstufen und den zu analysierenden Proben unbekannter Konzentration. Die entsprechenden Kalibrations- und Qualitätskontrollproben wurden in Serum angesetzt und zusammen mit den Blutserumproben unbekannten Gehaltes aufgearbeitet.

Die jeweils zum Meßzeitpunkt im Blutserum enthaltenden Mengen der Tramadolenantiomere und ggf. der Metabolite wurden unter Verwendung von Diazoethan als Derivatisierungsreagenz aufkonzentriert und die so erhaltenen Proben anschließend mittels stereoselektiver Gaschromatographie mit stickstoffselektiver Detektion analysiert.

Die Osmolalität der erfindungsgemäßen Darreichungsformen wurde durch Gefrierpunktserniedrigung nach Pharm. Eur. 97 gemäß Kapitel 2.2.35 bestimmt. Die entsprechende Literaturbeschreibung wird hiermit als Referenz eingeführt und gilt somit als Teil der Offenbarung. Die Messung erfolgte mittels eines Meßgerätes vom Typ M (Dr. H. Knauer KG, Berlin, Deutschland).

Die Kalibrierung wurde mit destilliertem Wasser für 0 mOsmol/kg sowie mit einer Eichlösung (Dr. H. Knauer KG, Berlin, Deutschland) oder alternativ 12,687 g Natriumchlorid, gelöst in 1 kg destilliertem Wasser für 400 mOsmol/kg durchgeführt.

Im folgenden wird die Erfindung anhand von Beispielen erläutert.

### Beispiele:

### Beispiel 1:

### 1. Stufe: Herstellung des Wirkstoffsalzes

Zur Herstellung des Wirkstoffsalzes aus Tramadol und Diclofenac wurden unter aseptischen Bedingungen äquimolare Mengen von Tramadolhydrochlorid und Diclofenac-Natrium jeweils in einer möglichst geringen Menge Wasser vollständig gelöst. Anschließend wurden beide Lösungen unter Rühren miteinander vermischt. Das Wirkstoffsalz kristallisierte beim Abkühlen aus der wäßrigen Lösung schon nach kurzer Zeit aus und wurde nach herkömmlichen Methoden isoliert und mit Ethanol gereinigt.

### 2. Stufe: Herstellung des Suspensionsmediums:

In einem autoklavierten Becherglas wurden unter Rühren bei Raumtemperatur (20 bis 25°C) in 251,97 g Wasser für Injektionszwecke nach Ph. Eur. (83,99 Gew.-%), 0,03 g Polysorbat 80 nach Ph. Eur. (0,01 Gew.-%), 3,00 g Saccharose nach Ph. Eur. (1,00 Gew.-%) und 45,00 g Macrogol 4000 nach Ph. Eur. (15,00 Gew.-%) gelöst. Nach vollständigem Auflösen aller festen Ausgangsstoffe in Wasser wurde die so erhaltene Lösung zur Entfernung von Verunreinigungen, wie z.B. Flusen oder nichtsichtbaren Partikeln, über einen Filter mit einer Porenweite von 5 µm (Millipore SVLP) filtriert. Anschließend wurde die filtrierte Lösung in einem verschlossenen Gefäß 30 Minuten lang bei einer Temperatur von 121 °C und einem Druck von 2 bar autoklaviert.

Die Osmolalität der so erhaltenen Lösung wurde nach der oben angegebenen Methode bestimmt und betrug 300 mOsm/kg.

### 3. Stufe: Herstellung der Darreichungsform

Das gemäß der 1. Stufe erhaltene Wirkstoffsalz wurde unter aseptischen Bedingungen gemörsert, so daß 98 % der Salzpartikel einen Partikeldurchmesser ≤ 30 µm aufwiesen. Anschließend wurden jeweils 293,5 mg des Salzes in je 5 ml des gemäß der 2. Stufe erhaltenen Suspensionsmediums suspendiert und in eine Injektionsflasche gefüllt. Zur gegebenenfalls notwendigen Resuspendierung wurden jeweils auch 3 bis 5 sterilisierte Glaskügelchen zugegeben und die Injektionsflaschen jeweils mit einem sterilisierten Brombutylgummistopfen verschlossen und verbördelt.

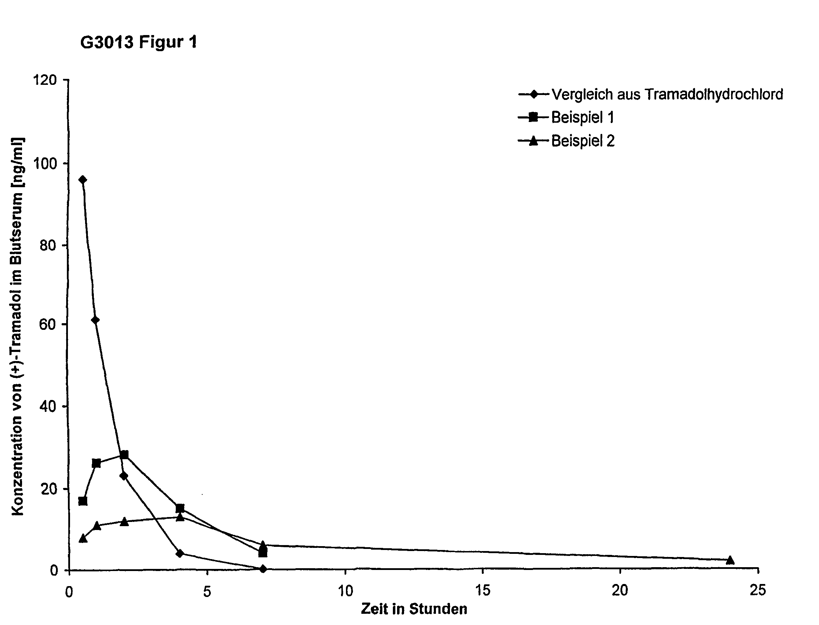

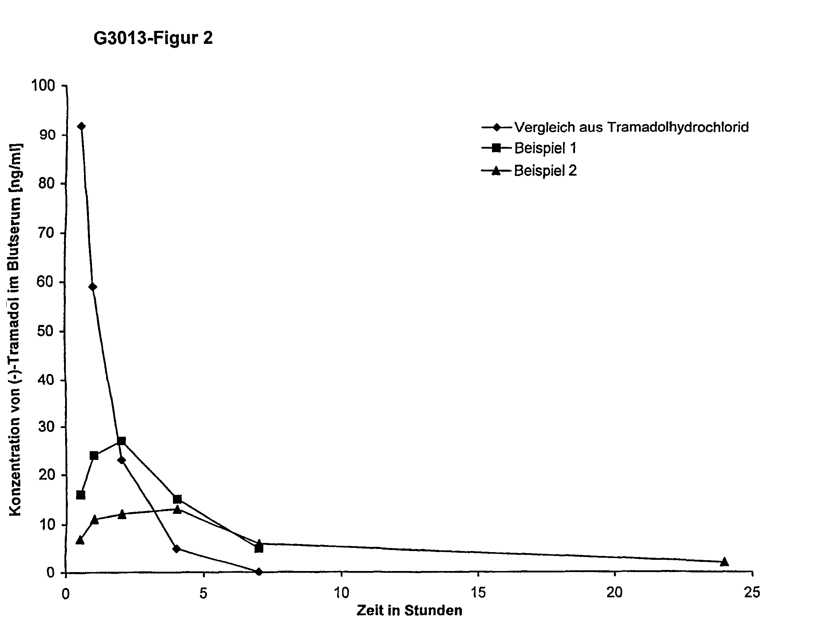

Die so erhaltene Darreichungsform wurde nach den obenstehend angegebenen Methode untersucht und die jeweiligen Konzentrationen von (+)-Tramadol und (-)-Tramadol im Blutserum bestimmt. Das Ergebnis dieser Untersuchungen für (+)-Tramadol und die Werte für die entsprechende Tramadolhydrochlorid-Darreichungsform sind in der Figur 1 wiedergegeben. Das Ergebnis für (-)-Tramadol und die entsprechenden Werte für die Tramadolhydrochlorid-Darreichungsform sind in Figur 2 wiedergegeben.

### Beispiel 2:

### 1. Stufe

### Herstellung des Suspensionsmediums:

Zur Entfernung von Verunreinigungen, wie beispielsweise Flusen oder nichtsichtbaren Partikeln, wurde Rizinusöl über einen Filter mit einer Porenweite von 5 µm (Millipore SVLP) filtriert. Anschließend wurde das filtrierte Öl in einem verschlossenen Gefäß bei 150 °C für eine Stunde sterilisiert.

### 2. Stufe:

### Herstellung der Darreichungsform

Das gemäß Beispiel 1, 1. Stufe hergestellte Wirkstoffsalz wurde unter aseptischen Bedingungen gemörsert, so daß 98 % der Salzpartikel einen Partikeldurchmesser ≤ 30 µm aufwiesen. Anschließend wurden jeweils 293,5 mg des Salzes in 5 ml des gemäß der 1. Stufe erhaltenen hydrophoben Suspensionsmediums suspendiert und in eine Injektionsflasche gefüllt. Zur gegebenenfalls notwendigen Resuspendierung wurden jeweils auch 3 bis 5 sterilisierte Glaskügelchen zugegeben, die vials mit sterilisierten Brombutylgummistopfen verschlossen und anschließend verbördelt.

Die so erhaltene Darreichungsform wurde nach den obenstehend angegebenen Methode untersucht und die jeweiligen Konzentrationen von (+)-Tramadol und (-)-Tramadol im Blutserum bestimmt. Das Ergebnis dieser Untersuchungen für (+)-Tramadol und die Werte für die entsprechende Tramadolhydrochlorid-Darreichungsform sind in der Figur 1 wiedergegeben. Das Ergebnis für (-)-Tramadol und die entsprechenden Werte für die Tramadolhydrochlorid-Darreichungsform sind in Figur 2 wiedergegeben.

## Patentansprüche

1. Parenteral applizierbare Darreichungsformen enthaltend eine Suspension des Salzes aus den Wirkstoffen Tramadol und Diclofenac.

2. Parenteral applizierbare Darreichungsformen gemäß Anspruch 1, **dadurch gekennzeichnet, daß** mindestens 95 % der suspendierten Salzpartikel einen Partikeldurchmesser im Bereich von ≤ 50 µm, vorzugsweise im Bereich von ≤ 30 µm, besonders bevorzugt im Bereich von ≤ 5 µm aufweisen.

3. Parenteral applizierbare Darreichungsformen gemäß Anspruch 1 oder 2, **dadurch gekennzeichnet, daß** das zu applizierende Volumen der Darreichungsformen ≤ 5 ml, vorzugsweise ≤ 4 ml, besonders bevorzugt ≤ 2 ml beträgt.

4. Parenteral applizierbare Darreichungsformen gemäß einem der Ansprüche 1 bis 3, **dadurch gekennzeichnet, daß** das Suspensionsmedium ein physiologisch verträgliches hydrophobes Suspensionsmedium ist.

5. Parenteral applizierbare Darreichungsformen gemäß Anspruch 4, **dadurch gekennzeichnet, daß** als hydrophobes Suspensionsmedium ein synthetisches, halbsynthetisches oder natürliches Öl oder ein Gemisch aus wenigstens zwei der vorstehend genannten Öle vorliegt.

6. Parenteral applizierbare Darreichungsformen gemäß Anspruch 5, **dadurch gekennzeichnet, daß** als Öl mittelkettige Triglyceride mit einer Kettenlänge von C₈ bis C₁₀ im Carbonsäure-Teil, Sojaöl, Sesamöl, Erdnußöl, Olivenöl, Kokosöl, Rizinusöl, Sonnenblumenöl, Distelöl oder die entsprechenden, hydrierten Öle oder ein Gemisch aus wenigstens zwei der vorstehend genannten Verbindungen, vorzugsweise Rhinzinusöl, gegebenenfalls in Kombination mit physiologisch verträglichen Antioxidantien, vorzugsweise Tocopherolen und/oder Tocopherolestern, butyliertem Hydroxyanisol und/oder butyliertem Hydroxytoluol vorliegt.

7. Parenteral applizierbare Darreichungsformen gemäß einem der Ansprüche 1 bis 3, **dadurch gekennzeichnet, daß** das Suspensionsmedium ein physiologisch verträgliches hydrophiles Suspensionsmedium ist.

8. Parenteral applizierbare Darreichungsformen gemäß Anspruch 7, **dadurch gekennzeichnet, daß** das hydrophile Suspensionsmedium auf Wasser basiert.

9. Parenteral applizierbare Darreichungsformen gemäß einem der Ansprüche 1 bis 8, **dadurch gekennzeichnet, daß** als weitere physiologisch verträgliche Hilfsstoffe pH-Regulatoren, Regulatoren zur Einstellung der Osmolalität, grenzflächenaktive Verbindungen, Viskositätsregulatoren, Peptisatoren, Puffer, Konservierungsmittel oder ein Gemisch aus wenigstens zwei Vertretern aus verschiedenen, vorstehend genannten Regulatorenklassen oder aus wenigstens zwei Vertretern aus einer Regulatorenklasse vorliegen.

10. Parenteral applizierbare Darreichungsformen gemäß Anspruch 9, **dadurch gekennzeichnet, daß** als grenzflächenaktiven Verbindungen Polyalkylenglykole, Phospholipide, Ether oder Ester von gesättigten oder ungesättigten Fettalkoholen oder Fettsäuren mit Polyalkylenglykolen, Polysorbate oder Gemische aus wenigstens zwei Vertretern aus verschiedenen, vorstehend genannten Verbindungsklassen oder aus wenigstens zwei Vertretern aus einer Regulatorenklasse vorliegen.

11. Parenteral applizierbare Darreichungsform gemäß Anspruch 10, **dadurch gekennzeichnet, daß** als Polyalkylenglykole Polyethylenglykole, Polypropylenglykole, Ethylenoxid, Propylenoxid-Blockcopolymere oder Gemische aus wenigstens zwei dieser Polyalkylenglykole vorliegen.

12. Parenteral applizierbare Darreichungsform gemäß Anspruch 10 oder 11, **dadurch gekennzeichnet, daß** Ether oder Ester von gesättigten oder ungesättigten Fettalkoholen oder Fettsäuren mit Polyethylenglykolen und/oder Polypropylenglykolen vorliegen.

13. Parenteral applizierbare Darreichungsformen gemäß einem der Ansprüche 10 bis 12, **dadurch gekennzeichnet, daß** als Polysorbate Mono-, Di-, oder Triester von gesättigten oder ungesättigten Fettsäuren und Sorbitol und/oder seinem Anhydrid, die bis zu 20 Mol Ethylenoxid-Einheiten pro Mol Sorbitol bzw. Anhydrid aufweisen können oder Gemische aus wenigstens zwei dieser Polysorbate vorliegen.

14. Parenteral applizierbare Darreichungsformen gemäß Anspruch 13, **dadurch gekennzeichnet, daß** als Fettsäure Ölsäure, Laurinsäure, Palmitinsäure oder Stearinsäure vorliegt.

15. Parenteral applizierbare Darreichungsformen gemäß Anspruch 13 oder 14, **dadurch gekennzeichnet, daß** als Polysorbat Polyethoxysorbitanmonolaurat mit 20 Ethylenoxid-Einheiten, Polyethoxysorbitanmonolaurat mit 4 Ethylenoxid-Einheiten, Polyethoxysorbitanmonopalmitat mit 20 Ethylenoxid-Einheiten, Polyethoxysorbitanmonostearat mit 20 Ethylenoxid-Einheiten, Polyethoxysorbitanmonostearat mit 4 Ethylenoxid-Einheiten, Polyethoxysorbitantristearat mit 20 Ethylenoxid-Einheiten, Polyethoxysorbitanmonooleat mit 20 Ethylenoxid-Einheiten, Polyethoxysorbitanmonooleat mit 5 Ethylenoxid-Einheiten oder Polyethoxysorbitantrioleat mit 20 Ethylenoxideinheiten oder ein Gemisch aus wenigstens zwei der vorstehend genannten Verbindungen vorliegt.

16. Parenteral applizierbare Darreichungsformen gemäß einem der Ansprüche 9 bis 15, **dadurch gekennzeichnet, daß** als Regulatoren zur Einstellung der Osmolalität wasserlösliche physiologisch verträgliche anorganische Salze, Zucker, Zuckeralkohole oder Polyalkylenglykole oder Gemische aus wenigstens zwei Vertretern aus verschiedenen, vorstehend genannten Regulatorenklassen oder aus wenigstens zwei Vertretern aus einer Regulatorenklasse vorliegen.

17. Parenteral applizierbare Darreichungsformen gemäß Anspruch 16, **dadurch gekennzeichnet, daß** das anorganische Salz ein Alkalisalz, vorzugsweise Natriumchlorid ist.

18. Parenteral applizierbare Darreichungsformen gemäß Anspruch 16 oder 17, **dadurch gekennzeichnet, daß** der Zucker Saccharose und/oder Dextrose ist.

19. Parenteral applizierbare Darreichungsformen gemäß einem der Ansprüche 16 bis 18, **dadurch gekennzeichnet, daß** der Zuckeralkohol Mannitol ist.

20. Parenteral applizierbare Darreichungsformen gemäß einem der Ansprüche 16 bis 19, **dadurch gekennzeichnet, daß** die Polyalkylenglykole Polyethylenglykole, vorzugsweise Polyethylenglykole mit einem Molekulargewicht von 1000 bis 8000 g/mol, besonders bevorzugt Polyethylenglykole mit einem Molekulargewicht von 2500 bis 5000 g/mol, sind.

21. Parenteral applizierbare Darreichungsformen gemäß einem der Ansprüche 9 bis 20, **dadurch gekennzeichnet, daß** als Konservierungsmittel 1,1,1-Trichlor-2-methyl-2-propanol, Phenylethylalkohol, Sorbinsäure, Benzylalkohol, Alkylbenzyldimethylammoniumchlorid mit einer Kettenlänge von C₈ bis C₁₈ im Alkylteil, m-Kresol, 4-Hydroxyalkylbenzoat oder ein Gemisch aus zwei oder mehr dieser vorstehend genannten Konservierungsmittel vorliegen.

22. Parenteral applizierbare Darreichungsformen gemäß Anspruch 21, **dadurch gekennzeichnet, daß** das 4-Hydroxyalkylbenzoat 4-Hydroxymethylbenzoat und/oder 4-Hydroxypropylbenzoat ist.

23. Parenterale Darreichungsformen gemäß einem der Ansprüche 9 bis 22, **dadurch gekennzeichnet, daß** das Suspensionsmedium Wasser ist, das
a) 0,001 bis 1 Gew.-%, vorzugsweise 0,0015 bis 0,1 Gew.-%, besonders bevorzugt 0,005 bis 0,015 Gew.-% physiologisch verträgliche grenzflächenaktive Verbindungen, vorzugsweise Polysorbate
b1) 3 bis 10 Gew.-% wenigstens eines physiologisch verträglichen Osmolalitäts-Regulators ausgewält aus der Gruppe Monosaccharide, Oligosaccharide und Zuckeralkohole, oder
b2) eine Mischung aus wenigstens einem der unter b1) genannten Osmolalitäts-Regulatoren in Mengen von 0,5 bis 5 Gew.-% und aus Polyethylenglykolen in Mengen von 10 bis 20 Gew.-%
jeweils bezogen auf das gesamte Suspensionsmedium einschließlich der Komponenten a) und b1) oder a) und b2) enthält.

24. Parenteral applizierbare Darreichungsform gemäß Anspruch 23, **dadurch gekennzeichnet, daß** als Polysorbate der Komponente a) mit Ölsäure, Laurinsäure, Palmitinsäure oder Stearinsäure einfach verestertes und mit Polyethylenglykol, vorzugsweise mit Polyethylenglykol mit einem Molekulargewicht von 1000 bis 6000 g/mol, besonders bevorzugt von 2500 bis 5000 g/mol verethertes Sorbitol und/oder dessen Anhydrid vorliegt.

25. Parenteral applizierbare Darreichungsform gemäß Anspruch 23 oder 24, **dadurch gekennzeichnet, daß** als Osmolalitäts-Regulator der Komponente b1) Saccharose und/oder Mannitol vorliegt.

26. Parenteral applizierbare Darreichungsform gemäß einem der Ansprüche 23 bis 25, **dadurch gekennzeichnet, daß** die Polyethylenglykole der Komponente b2) ein Molekulargewicht von 1000 bis 6000 g/mol, vorzugsweise von 2500 bis 5000 g/mol aufweisen.

27. Parenteral applizierbare Darreichungsformen gemäß einem der Ansprüche 1 bis 26 zur subkutanen oder intramuskulären Applikation.

## Claims

1. Dosage form which can be administered parenterally, comprising a suspension of the salt of the active ingredients tramadol and diclofenac.

2. Dosage form which can be administered parenterally according to Claim 1, **characterized in that** at least 95% of the suspended salt particles have a particle diameter in the range ≤ 50 *µ*m, preferably in the range ≤ 30 *µ*m, particularly preferably in the range ≤ 5 *µ*m.

3. Dosage form which can be administered parenterally according to Claim 1 or 2, **characterized in that** the volume of the dosage form to be administered is ≤ 5 ml, preferably ≤ 4 ml, particularly preferably ≤ 2 ml.

4. Dosage form which can be administered parenterally according to any of Claims 1 to 3, **characterized in that** the suspending medium is a physiologically tolerated hydrophobic suspending medium.

5. Dosage form which can be administered parenterally according to Claim 4, **characterized in that** a synthetic, semisynthetic or natural oil or a mixture of at least two of the aforementioned oils is present as hydrophobic suspending medium.

6. Dosage form which can be administered parenterally according to Claim 5, **characterized in that** medium chain length triglycerides with a chain length of from C₈ to C₁₀ in the carboxylic acid moiety, soybean oil, sesame oil, peanut oil, olive oil, coconut oil, castor oil, sunflower oil, safflower oil or the corresponding hydrogenated oils or a mixture of at least two of the aforementioned compounds, preferably castor oil, where appropriate in combination with physiologically tolerated antioxidants, preferably tocopherols and/or tocopherol esters, butylated hydroxyanisole and/or butylated hydroxytoluene, is present as oil.

7. Dosage form which can be administered parenterally according to any of Claims 1 to 3, **characterized in that** the suspending medium is a physiologically tolerated hydrophilic suspending medium.

8. Dosage form which can be administered parenterally according to Claim 7, **characterized in that** the hydrophilic suspending medium is based on water.

9. Dosage form which can be administered parenterally according to any of Claims 1 to 8, **characterized in that** pH regulators, regulators for adjusting the osmolality, surface-active compounds, viscosity regulators, peptizers, buffers, preservatives or a mixture of at least two representatives of various aforementioned classes of regulators or of at least two representatives of one class of regulators are present as further physiologically tolerated excipients.

10. Dosage form which can be administered parenterally according to Claim 9, **characterized in that** polyalkylene glycols, phospholipids, ethers or esters of saturated or unsaturated fatty alcohols or fatty acids with polyalkylene glycols, polysorbates or mixtures of at least two representatives of various aforementioned classes of compounds or of at least two representatives of one class of regulators are present as surface-active compounds.

11. Dosage form which can be administered parenterally according to Claim 10, **characterized in that** polyethylene glycols, polypropylene glycols, ethylene oxide, propylene oxide block copolymers or mixtures of at least two of these polyalkylene glycols are present as polyalkylene glycols.

12. Dosage form which can be administered parenterally according to Claim 10 or 11, **characterized in that** ethers or esters of saturated or unsaturated fatty alcohols or fatty acids with polyethylene glycols and/or polypropylene glycols are present.

13. Dosage form which can be administered parenterally according to any of Claims 10 to 12, **characterized in that** mono-, di- or triesters of saturated or unsaturated fatty acids and sorbitol and/or its anhydride, each of which may have up to 20 mol of ethylene oxide units per mole of sorbitol or anhydride, or mixtures of at least two of these polysorbates are present as polysorbates.

14. Dosage form which can be administered parenterally according to Claim 13, **characterized in that** oleic acid, lauric acid, palmitic acid or stearic acid is present as fatty acid.

15. Dosage form which can be administered parenterally according to Claim 13 or 14, **characterized in that** polyethoxysorbitan monolaurate with 20 ethylene oxide units, polyethoxysorbitan monolaurate with 4 ethylene oxide units, polyethoxysorbitan monopalmitate with 20 ethylene oxide units, polyethoxysorbitan monostearate with 20 ethylene oxide units, polyethoxysorbitan monostearate with 4 ethylene oxide units, polyethoxysorbitan tristearate with 20 ethylene oxide units, polyethoxysorbitan monooleate with 20 ethylene oxide units, polyethoxysorbitan monooleate with 5 ethylene oxide units or polyethoxysorbitan trioleate with 20 ethylene oxide units, or a mixture of at least two of the aforementioned compounds, is present as polysorbate.

16. Dosage form which can be administered parenterally according to any of Claims 9 to 15, **characterized in that** water-soluble physiologically tolerated inorganic salts, sugars, sugar alcohols or polyalkylene glycols or mixtures of at least two representatives of different aforementioned classes of regulators or of at least two representatives of one class of regulators are present as regulators.

17. Dosage form which can be administered parenterally according to Claim 16, **characterized in that** the inorganic salt is an alkali metal salt, preferably sodium chloride.

18. Dosage form which can be administered parenterally according to Claim 16 or 17, **characterized in that** the sugar is sucrose and/or dextrose.

19. Dosage form which can be administered parenterally according to any of Claims 16 to 18, **characterized in that** the sugar alcohol is mannitol.

20. Dosage form which can be administered parenterally according to any of Claims 16 to 19, **characterized in that** the polyalkylene glycols are polyethylene glycols, preferably polyethylene glycols having a molecular weight of from 1 000 to 8 000 g/mol, particularly preferably polyethylene glycols having a molecular weight of from 2 500 to 5 000 g/mol.

21. Dosage form which can be administered parenterally according to any of Claims 9 to 20, **characterized in that** 1,1,1-trichloro-2-methyl-2-propanol, phenylethyl alcohol, sorbic acid, benzyl alcohol, alkylbenzyldimethylammonium chloride having a chain length of from C₈ to C₁₈ in the alkyl moiety, m-cresol, 4-hydroxyalkylbenzoate or a mixture of two or more of these aforementioned preservatives are present as preservatives.

22. Dosage form which can be administered parenterally according to Claim 21, **characterized in that** the 4-hydroxyalkylbenzoate is 4-hydroxymethylbenzoate and/or 4-hydroxypropylbenzoate.

23. Parenteral dosage form according to any of Claims 9 to 22, **characterized in that** the suspending medium is water which comprises
a) 0.001 to 1% by weight, preferably 0.0015 to 0.1% by weight, particularly preferably 0.005 to 0.015% by weight of physiologically tolerated surface-active compounds, preferably polysorbates,
b1) 3 to 10% by weight of at least one physiologically tolerated osmolality regulator selected from the group of monosaccharides, oligosaccharides and sugar alcohols, or
b2) a mixture of at least one of the osmolality regulators mentioned under b1) in amounts of from 0.5 to 5% by weight and of polyethylene glycols in amounts of from 10 to 20% by weight
in each case based on the complete suspending medium including components a) and b1) or a) and b2).

24. Dosage form which can be administered parenterally according to Claim 23, **characterized in that** sorbitol and/or its anhydride which is monoesterified with oleic acid, lauric acid, palmitic acid or stearic acid and etherified with polyethylene glycol, preferably with polyethylene glycol having a molecular weight of from 1 000 to 6 000 g/mol, particularly preferably from 2 500 to 5 000 g/mol, is present as polysorbates of component a).

25. Dosage form which can be administered parenterally according to Claim 23 or 24, **characterized in that** sucrose and/or mannitol is present as osmolality regulator of component b1).

26. Dosage form which can be administered parenterally according to any of Claims 23 to 25, **characterized in that** the polyethylene glycols of component b2) have a molecular weight of from 1 000 to 6 000 g/mol, preferably from 2 500 to 5 000 g/mol.

27. Dosage form which can be administered parenterally according to any of Claims 1 to 26 for subcutaneous or intramuscular administration.

## Revendications

1. Formes pharmaceutiques administrables par voie parentérale contenant une suspension du sel des principes actifs tramadol et diclofénac.

2. Formes pharmaceutiques administrables par voie parentérale selon la revendication 1, **caractérisées en ce qu'**au moins 95 % des particules de sel en suspension ont une granulométrie ≤ 50 *µ*m, de préférence ≤ 30 *µ*m et tout particulièrement ≤ 5 *µ*m.

3. Formes pharmaceutiques administrables par voie parentérale selon la revendication 1 ou 2, **caractérisées en ce que** le volume des formes pharmaceutiques devant être appliqué est ≤ 5 ml, de préférence ≤ 4 ml, plus particulièrement ≤ 2 ml.

4. Formes pharmaceutiques administrables par voie parentérale selon l'une des revendications 1 à 3, **caractérisées en ce que** le milieu de suspension est un milieu de suspension hydrophobe toléré d'un point de vue physiologique.

5. Formes pharmaceutiques administrables par voie parentérale selon la revendication 4, **caractérisées en ce que** le milieu de suspension hydrophobe est une huile synthétique, semi-synthétique ou naturelle, ou un mélange d'au moins deux des huiles mentionnées ci-dessus.

6. Formes pharmaceutiques administrables par voie parentérale selon la revendication 5, **caractérisées en ce qu'**elles contiennent en tant qu'huile des triglycérides à longueur moyenne de chaîne, ayant une longueur de chaîne en C₈ à C₁₀ dans la partie acide carboxylique, l'huile de soja, l'huile de sésame, l'huile d'arachide, l'huile d'olive, l'huile de coprah, l'huile de ricin, l'huile de tournesol, l'huile de chardon ou les huiles hydrogénées correspondantes, ou un mélange d'au moins deux des composés mentionnés ci-dessus, de préférence l'huile de ricin, éventuellement en combinaison avec des antioxydants tolérés d'un point de vue physiologique, de préférence les tocophérols et/ou les esters du tocophérol, l'hydroxyanisole butylé et/ou l'hydroxytoluène butylé.

7. Formes pharmaceutiques administrables par voie parentérale selon l'une des revendications 1 à 3, **caractérisées en ce que** le milieu de suspension est un milieu de suspension hydrophile, toléré d'un point de vue physiologique.

8. Formes pharmaceutiques administrables par voie parentérale selon la revendication 7, **caractérisées en ce que** le milieu de suspension hydrophile est à base d'eau.

9. Formes pharmaceutiques administrables par voie parentérale selon l'une des revendications 1 à 8, **caractérisées en ce qu'**elles contiennent en tant qu'autres adjuvants tolérés d'un point de vue physiologique des régulateurs de pH, des régulateurs destinés à ajuster l'osmolalité, des composés tensioactifs, des régulateurs de viscosité, des agents de peptisation, des tampons, des conservateurs ou un mélange d'au moins deux représentants de classes différentes de régulateurs, mentionnées ci-dessus, ou d'au moins deux représentants d'une classe de régulateurs.

10. Formes pharmaceutiques administrables par voie parentérale selon la revendication 9, **caractérisées en ce qu'**elles contiennent en tant que composés tensioactifs des polyalkylèneglycols, des phospholipides, des éthers ou des esters d'alcools gras ou d'acides gras saturés ou insaturés avec des polyalkylèneglycols, des polysorbates, ou des mélanges d'au moins deux représentants de classes de composés différentes, mentionnées ci-dessus, ou d'au moins deux représentants d'une classe de régulateurs.

11. Forme pharmaceutique administrable par voie parentérale selon la revendication 10, **caractérisée en ce qu'**elle contient en tant que polyalkylèneglycols des polyéthylèneglycols, des polypropylèneglycols, de l'oxyde d'éthylène, des copolymères à blocs oxyde de propylène, ou des mélanges d'au moins deux de ces polyalkylèneglycols.

12. Forme pharmaceutique administrable par voie parentérale selon la revendication 10 ou 11, **caractérisée en ce qu'**elle contient des éthers ou des esters d'alcools gras ou d'acides gras saturés ou insaturés avec des polyéthylèneglycols et/ou des polypropylèneglycols.

13. Formes pharmaceutiques administrables par voie parentérale selon l'une des revendications 10 à 12, **caractérisées en ce qu'**elles contiennent en tant que polysorbates des mono-, di- ou triesters d'acides gras saturés ou insaturés, et du sorbitol et/ou de son anhydride, qui peuvent contenir jusqu'à 20 moles de motifs d'oxyde d'éthylène par mole de sorbitol ou d'anhydride, ou des mélanges d'au moins deux de ces polysorbates.

14. Formes pharmaceutiques administrables par voie parentérale selon la revendication 13, **caractérisées en ce qu'**elles contiennent en tant qu'acide gras l'acide oléique, l'acide laurique, l'acide palmitique ou l'acide stéarique.

15. Formes pharmaceutiques administrables par voie parentérale selon la revendication 13 ou 14, **caractérisées en ce qu'**elles contiennent en tant que polysorbate le monolaurate de sorbitan polyéthoxylé à 20 motifs oxyde d'éthylène, le monolaurate de sorbitan polyéthoxylé à 4 motifs oxyde d'éthylène, le monopalmitate de sorbitan polyéthoxylé à 20 motifs oxyde d'éthylène, le monostéarate de sorbitan polyéthoxylé à 20 motifs oxyde d'éthylène, le monostéarate de sorbitan polyéthoxylé à 4 motifs oxyde d'éthylène, le tristéarate de sorbitan polyéthoxylé à 20 motifs oxyde d'éthylène, le monooléate de sorbitan polyéthoxylé à 20 motifs oxyde d'éthylène, le monooléate de sorbitan polyéthoxylé à 5 motifs oxyde d'éthylène ou le trioléate de sorbitan polyéthoxylé à 20 motifs oxyde d'éthylène, ou un mélange d'au moins deux des composés mentionnés ci-dessus.

16. Formes pharmaceutiques administrables par voie parentérale selon l'une des revendications 9 à 15, **caractérisées en ce qu'**elles contiennent en tant que régulateurs destinés à ajuster l'osmolalité des sels inorganiques des sucres, des alcools de sucre ou des polyalkylèneglycols insolubles dans l'eau, tolérés d'un point de vue physiologique, ou des mélanges d'au moins deux représentants de classes de régulateurs différentes, mentionnées ci-dessus, ou d'au moins deux représentants d'une classe de régulateurs.

17. Formes pharmaceutiques administrables par voie parentérale selon la revendication 16, **caractérisées en ce que** le sel inorganique est un sel d'un métal alcalin, de préférence le chlorure de sodium.

18. Formes pharmaceutiques administrables par voie parentérale selon la revendication 16 ou 17, **caractérisées en ce que** le sucre est le saccharose et/ou le dextrose.

19. Formes pharmaceutiques administrables par voie parentérale selon l'une des revendications 16 à 18, **caractérisées en ce que** l'alcool de sucre est le mannitol.

20. Formes pharmaceutiques administrables par voie parentérale selon l'une des revendications 16 à 19, **caractérisées en ce que** les polyalkylèneglycols sont des polyéthylèneglycols, de préférence des polyéthylèneglycols ayant une masse moléculaire de 1000 à 8000 g/mol, plus particulièrement des polyéthylèneglycols ayant une masse moléculaire de 2500 à 5000 g/mol.

21. Formes pharmaceutiques administrables par voie parentérale selon l'une des revendications 9 à 20, **caractérisées en ce qu'**elles contiennent en tant que conservateur du 1,1,1-trichloro-2-méthyl-2-propanol, de l'alcool phényléthylique, de l'acide sorbique, de l'alcool benzylique, un chlorure d'alkylbenzyldiméthylammonium ayant une longueur de chaîne en C₈ à C₁₈ dans la partie alkyle, du m-crésol, un 4-hydroxyalkylbenzoate, ou un mélange d'au moins deux des conservateurs mentionnés ci-dessus.

22. Formes pharmaceutiques administrables par voie parentérale selon la revendication 21, **caractérisées en ce que** le 4-hydroxyalkylbenzoate est un 4-hydroxyméthylbenzoate et/ou un 4-hydroxypropylbenzoate.

23. Formes pharmaceutiques administrables par voie parentérale selon l'une des revendications 9 à 22, **caractérisées en ce que** le milieu de suspension est l'eau, qui contient
a) 0,001 à 1 % en poids, de préférence 0,0015 à 0,1 % en poids, plus particulièrement 0,005 à 0,015 % en poids de composés tensioactifs tolérés d'un point de vue physiologique, de préférence des polysorbates,
b1) 3 à 10 % en poids d'au moins un régulateur d'osmolalité toléré d'un point de vue physiologique, choisi dans le groupe des monosaccharides, des oligosaccharides et des alcools de sucre, ou
b2) un mélange d'au moins l'un des régulateurs d'osmolalité mentionnés en b1), en des quantités de 0,5 à 5 % en poids, et de polyéthylèneglycols en des quantités de 10 à 20 % en poids,
dans tous les cas par rapport à la totalité du milieu de suspension, y compris les composants a) et b1) ou a) et b2).

24. Forme pharmaceutique administrable par voie parentérale selon la revendication 23, **caractérisée en ce qu'**elle contient en tant que polysorbates du composant a) un sorbitol et/ou son anhydride, une fois estérifié avec l'acide oléique, l'acide laurique, l'acide palmitique ou l'acide stéarique, et éthérifié avec un polyéthylèneglycol, de préférence un polyéthylèneglycol ayant une masse moléculaire de 1000 à 6000 g/mol, plus particulièrement de 2500 à 5000 g/mol.

25. Forme pharmaceutique administrable par voie parentérale selon la revendication 23 ou 24, **caractérisée en ce qu'**elle contient en tant que régulateur d'osmolalité du composant b1) du saccharose et/ou du mannitol.

26. Forme pharmaceutique administrable par voie parentérale selon l'une des revendications 23 à 25, **caractérisée en ce que** les polyéthylèneglycols du composant b2) ont une masse moléculaire de 1000 à 6000 g/mol, de préférence de 2500 à 5000 g/mol.

27. Formes pharmaceutiques administrables par voie parentérale selon l'une des revendications 1 à 26 pour administration sous-cutanée ou intramusculaire.
